# NEUE EUROPÄISCHE PATENTSCHRIFT

(11) **EP 1 562 585 B2**
(45) Veröffentlichungstag und Bekanntmachung des Hinweises auf die Entscheidung über den Einspruch: **24.05.2017**
(45) Hinweis auf die Patenterteilung: 24.11.2010
(21) Anmeldenummer: 03769477.5
(22) Anmeldetag: 30.10.2003
(51) Int. Cl.: A61K 8/49

(54) **MELATONIN-TAGESDOSIERUNGSEINHEITEN**
DAILY MELATONIN DOSING UNITS
UNITE D'ADMINISTRATION QUOTIDIENNE D'UNE DOSE DE MELATONINE

(30) Priorität: 30.10.2002 DE 10250646; 18.11.2002 DE 20217814 U; 29.01.2003 US 353056
(43) Veröffentlichungstag der Anmeldung: 17.08.2005
(73) Patentinhaber: Asat AG Applied Science & Technology, 6300 Zug (CH)
(72) Erfinder: SCHMID, Hans, W., CH-6303 Zug (CH)
(74) Vertreter: Weickmann & Weickmann PartmbB
(86) Internationale Anmeldenummer: PCT/EP2003/012099
(87) Internationale Veröffentlichungsnummer: WO 2004/039369

(56) Entgegenhaltungen:
- EP-A- 0 867 181
- EP-A1- 0 150 751
- WO-A1-99/32072
- AT-B- 367 634
- DE-A- 10 110 418
- DE-U1- 20 019 365
- GB-A- 2 079 238
- US-A- 4 746 674
- US-A- 5 702 691
- US-A- 5 985 293
- US-B1- 6 281 241
- CLINICAL STUDY REPORT MEL-COS-1 ASATONA AG 01 September 2003,
- CLINICAL STUDY REPORT MEL-ALO-2 ASATONA AG 09 September 2004,
- USE TEST MEL-COS-AS05 ASATONA AG 05 Juni 2008,
- NEUE ZÜRCHER ZEITUNG Nr. 208, 08 September 2010, Seite 58

## Beschreibung

Die vorliegende Erfindung betrifft einen Einwegbehälter für ein Arzneimittel oder kosmetisches Mittel zur topischen Applikation, enthaltend eine Einzeldosis an Melatonin oder einem Melatonin-Derivat, die einer lokal wirksamen Dosis entspricht, die aber keine systemische Wirkung hervorruft, wie in den Ansprüchen definiert.

Melatoninhaltige Mittel können für unterschiedliche medizinische und kosmetische Zwecke verwendet werden. Das Dokument DE 101 10 418.9 offenbart beispielsweise melatoninhaltige Zusammensetzungen für eine topische Anwendung auf der Haut und im Haar.

EP 0 867 181 A1 offenbart eine nasal verabreichbare Zusammensetzung, welche Melatonin sowie ein Additiv ausgewählt aus Glycerin, Cyclodextrin oder Gemischen hiervon umfasst und zur Schlafinduktion verwendet wird. Die Zusammensetzung wird in Mehrwegbehältern bereitgestellt, welche beispielsweise 100 Dosierungseinheiten von jeweils 0,2 mg oder 0,4 mg an Melatonin enthalten können, wobei Tagesdosen im Bereich von 0,05-1 mg an Melatonin eingesetzt werden.

US 6,281,241 B1 betrifft die Verwendung von Melatonin und melatoninhaltigen Zusammensetzungen zur Behandlung der androgenetischen Alopezie vom weiblichen Typ. Die Zusammensetzung wird in lichtgeschützten Aluminiumflaschen bereitgestellt, welche jeweils 33 ml einer 0,1 %igen Lösung von Melatonin in einem Ethanol/Wasser-Gemisch beinhalten. Die verabreichte Tagesdosis an Melatonin liegt im Bereich von 0.001-10 mg.

US 4,746,674 beschreibt ein Verfahren zur topischen Behandlung der Haut und/oder Kopfhaut, welches das Verabreichen einer Melatonin-haltigen Zusammensetzung umfasst, wobei Melatonin in einer Menge von 10-4 bis etwa 1 Gew.-% zur Anwendung gelangt. Die verabreichte Tagesdosis liegt im Fall einer oralen Applikation im Bereich von 0,1 bis etwa 100 mg/kg.

US 5,985,293 betrifft eine topisch applizierbare kosmetische Zusammensetzung zur Verbesserung oder Aufrechterhaltung des Erscheinungsbildes der Haut und/oder Kopfhaut, welche Melatonin oder ein Melatonin-Analogon in einer Menge von weniger als 10⁻⁴ Gew.-%, bezogen auf das Gesamtgewicht der Zusammensetzung, umfasst.

EP 0 150 751 A1 offenbart einen Einwegbehälter zur topischen Applikation eines Präparats, welcher einen Behälterkörper mit flachen, sich quer erstreckenden und gegenüberliegend angeordneten, flossenartigen Teilen sowie einen sich axial erstreckenden Hals umfasst, wobei der Hals eine Sollbruchstelle zur Abgabe des in dem Einwegbehälter enthaltenen Präparats aufweist.

GB 2 079 238 A betrifft einen Dispenser oder Behälter zur topischen Applikation eines Arzneimittels, welcher aus Kunststoff gebildet ist und eine Einzeldosis von beispielsweise 0,5 bis 5,0 g des Arzneimittels enthält. Der Behälter besitzt einen Auslass, welcher mittels eines Verschlusses, der von dem Rest des Behälters über eine Sollbruchstelle abgetrennt werden kann, verschlossen ist.

AT 367 634 B offenbart eine Mehrfachpackung bestehend aus allseitig verschlossenen einzelnen Behältern für Flüssigkeiten und/oder Feststoffe, die in einer Ebene nebeneinander angeordnet und miteinander verbunden sind, wobei die einzelnen Behälter jeweils an einer Schmalseite durch einen in oder parallel zur Behälterebene liegenden Steg integral miteinander verbunden sind, dessen Querschnitt an den Verbindungsstellen mit den Behältern verjüngt ist.

DE 200 19 365 U1 offenbart Einwegbehälter zur Aufnahme von flüssigen oder gelartigen pharmazeutischen Präparaten, welche eine Einzeldosis des pharmazeutischen Präparats enthalten und einen Formkörper, eine abdrehbare und/oder abreissbare und/oder aufbrechbare Verschlusskappe sowie eine an der Unterseite des Formkörpers befindliche Fahne zur Anbringung von Prägungen und/oder Etiketten umfassen. In einer bevorzugten Ausführungsform werden die aus Kunststoff bestehenden Einwegbehälter in Blöcken aus 5-15 einzelnen Behältern bereitgestellt.

Der Gegenstand der vorliegenden Erfindung betrifft einen Einwegbehälter, der ein Arzneimittel oder kosmetisches Mittel zur topischen Applikation enthält, enthaltend eine Einzeldosis an Melatonin oder einem Melatonin-Derivat, die einer lokal wirksamen Dosis entspricht, die aber keine systemische Wirkung hervorruft, wobei die Einzeldosis von 0,01 bis 0,2 mg Melatonin oder Melatonin-Derivat beträgt und die Einzeldosis einer Tagesdosis entspricht, wobei das Mittel als Wirkstoffe eine Kombination von Melatonin oder einem Melatonin-Derivat mit Biotin und Gingko Biloba enthält.

Der erfindungsgemäße Einwegbehälter ermöglicht die Applikation einer Dosis an Melatonin oder einem Melatonin-Derivat, die lokal wirksam ist, jedoch keine unerwünschte Veränderung des normalen Melatoninplasmaspiegels verursacht. Die erfindungsgemäß applizierte Einzeldosis an Melatonin oder einem Melatonin-Derivat verursacht beispielsweise keine signifikante Erhöhung des Melatoninplasmaspiegels.

Der Begriff "Melatonin oder Melatonin-Derivat", wie er im Rahmen dieser Anmeldung verwendet wird, umfasst Melatonin oder/und Melatonin-Derivate sowie Salze, Ester oder Komplexverbindungen davon. Bevorzugte Melatonin-Derivate umfassen beispielsweise 5-Methoxytryptamin, 5-Methoxytryptophan, 5-Methoxytryptophol, 5-Methoxyindol-3-essigsäure und 6-Hydroxymelatonin.

Die erfindungsgemäße Einzeldosis stellt eine Dosis dar, welche eine positive kosmetische oder/und therapeutische Wirkung ohne signifikante Veränderungen des Plasmaspiegels ermöglicht. Erfindungsgemäß beträgt die Einzeldosis von etwa 0,01 bis etwa 0,2 mg Melatonin. Die Einzeldosis entspricht einer Tagesdosis, bevorzugt einer abends anzuwendenden Tagesdosis.

Das Arzneimittel oder kosmetische Mittel liegt bevorzugt als flüssige Formulierung vor. Für eine topische Applikation des Mittels sind beispielsweise Lösungen, Suspensionen, Emulsionen, Mikroemulsionen, Nanosysteme, Cremes, Gele, Lotionen, Sprays, Schäume oder Salben geeignet sowie jede andere Formulierung, welche topisch appliziert werden kann. Besonders bevorzugt liegt das Mittel in Form einer kosmetischen Lösung vor.

Das Arzneimittel oder kosmetische Mittel, welches im erfindungsgemäßen Einwegbehälter enthalten ist, kann, je nach Verwendungszweck, verschiedene Konzentrationen an Melatonin oder einem Melatonin-Derivat aufweisen. Bevorzugt enthält das Mittel eine Konzentration von etwa 0,001 bis etwa 0,01 % (Gew.), mehr bevorzugt von etwa 0,003 bis 0,004 % (Gew.) an Melatonin oder einem Melatonin-Derivat. Eine besonders bevorzugte Konzentration beträgt etwa 0,0033 % (Gew.).

Das Arzneimittel oder kosmetische Mittel enthält eine Kombination der Wirkstoffe Melatonin oder Melatonin-Derivat mit Biotin und Gingko Biloba. Die Wirkstoffe in dieser Ausführungsform können in verschiedenen Dosierungen vorliegen. Eine besonders bevorzugte Dosierung dieser Kombination beträgt von etwa 0,05 bis etwa 0,2 mg, besonders bevorzugt etwa 0,1 mg Melatonin oder Melatonin-Derivat, von etwa 0,2 bis etwa 0,4 mg, besonders bevorzugt etwa 0,3 mg Biotin sowie von etwa 1,3 bis etwa 1,7 mg, besonders bevorzugt etwa 1,5 mg Gingko Biloba. Gingko Biloba kann hierbei beispielsweise als Extrakt, insbesondere als Trockenextrakt oder/und in Form eines oder mehrerer Inhaltsstoffe enthalten sein.

Weiterhin kann das Arzneimittel oder kosmetische Mittel einen oder mehrere kosmetische oder/und pharmazeutische Hilfs- bzw. Zusatzstoffe enthalten, wie beispielsweise Verdickungsmittel, Mineralstoffe oder Duftstoffe.

Die erfindungsgemäße Zusammensetzung kann zur Förderung des Haarwachstums eingesetzt werden, insesondere zur Prävention oder/und Behandlung von Alopezie bei Männern oder bei Frauen. Besonders bevorzugte Indikationen sind die androgenetische Alopezie des männlichen Typs, die androgenetische Alopezie des weiblichen Typs, die diffuse Alopezie des männlichen Typs und die diffuse Alopezie des weiblichen Typs.

Der erfindungsgemäße Einwegbehälter kann je nach beabsichtigter Applikation bzw. Verwendungszweck unterschiedliche Volumina des Arzneimittels oder kosmetischen Mittels enthalten. In einer bevorzugten Ausführungsform enthält der Einwegbehälter etwa 2,5 bis etwa 3,5 ml, bevorzugt etwa 2,9 bis etwa 3,2 ml des Mittels. Ein besonders bevorzugtes Volumen beträgt etwa 3,0 ml.

Das therapeutische oder kosmetische Mittel kann durch jedes geeignete Verfahren, das im Stand der Technik bekannt ist, abgefüllt sein. Bevorzugt ist das Mittel unter GMP-Bedingungen abgefüllt. Dadurch kann gewährleistet werden, dass das Mittel steril ist und sich bei einer Applikation des Mittels keine Probleme etwa im Hinblick auf bakterielle Kontamination ergeben.

Der erfindungsgemäße Einwegbehälter kann im Wesentlichen aus jedem beliebigen Material bestehen, insbesondere einem Material, das für die Verpakkung von Lebensmitteln und pharmazeutischen Produkten zugelassen ist. Bevorzugte Materialien sind beispielsweise Kunststoffe bzw. Gemische aus Kunststoffen. Besonders geeignete Kunststoffe sind beispielsweise Polyethylen, Polyvinylchlorid, Polystyrol, Polypropylen, Polycycloolefine oder Mischungen bzw. Copolymere davon, besonders bevorzugt sind Polyethylen oder Polycycloolefine.

Der Einwegbehälter kann je nach erwünschter optischer Erscheinung transparent, nicht transparent, farblos oder farbig sein. Dem Kunststoff können beispielsweise beliebige Farbstoffe beigemischt sein, um einen farbigen Behälter zu erhalten. Ein nicht transparenter Behälter wird bevorzugt dann verwendet, wenn das Mittel lichtempfindliche Substanzen enthält, da durch einen nicht transparenten Behälter zudem ein Lichtschutz erzielt werden kann.

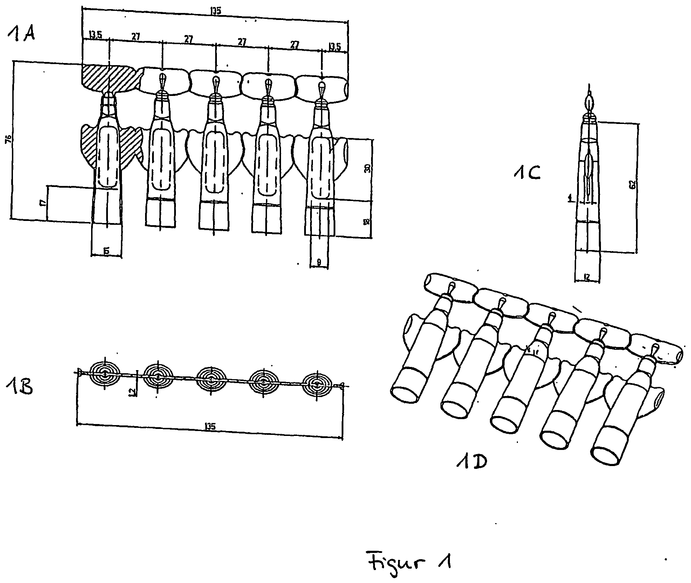

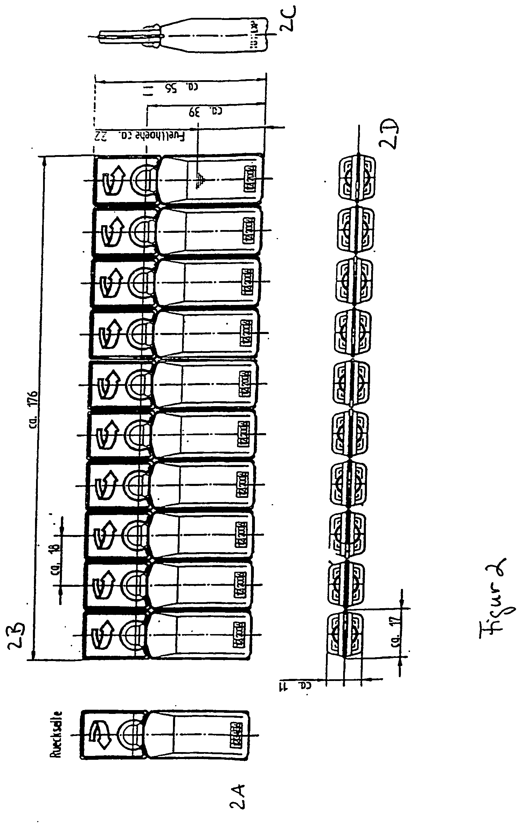

Der erfindungemäße Einwegbehälter kann eine im Wesentlichen beliebige Form aufweisen. Beispiele für bevorzugte Behälter sind in den Figuren 1 und 2 veranschaulicht. Bevorzugt umfasst ein geeigneter Einwegbehälter einen Teil, in welchem das Mittel enthalten ist, und einen Einwegverschluss, welcher durch eine Sollbruchstelle mit dem unteren Teil verbunden ist.

Vorteilhafterweise wird eine einfache und sichere Handhabung bei der Verwendung des Behälters dadurch gewährleistet, dass er bevorzugt einen durch Drehen zu öffnenden Einwegverschluss an seinem Kopf aufweist. Somit kann der Behälter leicht geöffnet werden. Darüber hinaus kann das Mittel nach Öffnen nur bei Anwendung von Druck austreten. Dieser Druck kann beispielsweise beim Auftragen durch die Hand angewendet werden. Diese Eigenschaft ermöglicht es dem Anwender, das Mittel gezielt zu platzierten und gut zu dosieren. Wenn eine Applikation des Mittels auf die Kopfhaut bzw. die Haare erfolgen soll, kann der geöffnete Behälter wie ein Kamm direkt durch die Haare geführt werden. Dies ermöglicht eine leichte und sichere Handhabung des Behälters sowie eine richtige Dosierung des Mittels.

Je nach Verwendungszweck ist der Einwegbehälter durch eine Prägung oder Bedruckung beschriftet oder/und mit einem Etikett versehen.

Zwei oder mehrere Einwegbehälter können weiterhin miteinander trennbar verbunden sein. Bevorzugt sind 5 oder 10 Behälter miteinander verbunden und bilden eine Anordnung. Weiterhin kann ein Behälter oder eine Anordnung von Behältern verpackt sein, bevorzugt in eine lichtdichte Verpackung, besonders bevorzugt beispielsweise in einen Aluminiumbeutel.

Mit dem Begriff "Aluminiumbeutel" wie er hierin verwendet wird, sind sowohl Beutel aus Aluminium als auch Beutel, welche beispielsweise an der Außen- oder Innenfläche mit Aluminium beschichtet sind, umfasst. Beispielsweise können Beutel aus einer oder mehreren Schichten von Kunststoff oder/und Papier mit Aluminium beschichtet sein. Ein bevorzugter Beutel umfasst z.B. eine Aluminium enthaltende, laminierte Folie, welche zusätzlich Polyethylen, Polyester oder/und Papier umfasst.

Eine Verpackung, insbesondere eine lichtdichte Verpackung, ermöglicht eine Lagerung über einen längeren Zeitraum, ohne Verlust der Wirksamkeit des Mittels. Weiterhin kann eine solche Verpackung einen Schutz vor Gasverlust bereitstellen.

Je nach Verwendungszweck kann ein Aluminiumbeutel, in welchen ein oder mehrere Einwegbehälter verpackt sind, bedruckt oder/und mit einem Etikett versehen sein.

In einer weiteren Ausführungsform der Erfindung wird eine Verpackungseinheit, die mehrere Anordnungen von Einwegbehältern umfasst, bereitgestellt.

Die Erfindung wird weiterhin durch die beiliegenden Figuren 1 und 2 und Beispiele erläutert.
- **Fig. 1**: zeigt eine Anordnung von 5 trennbar verbundenen Einwegbehältern, welche eine bevorzugte Gestaltung aufweisen. **1 A** zeigt eine Vorderansicht, **1 B** eine Draufsicht, **1 C** eine Seitenansicht und **1 D** eine perspektive Ansicht schräg von oben.
- **Fig. 2 A**: zeigt eine Vorderansicht eines einzelnen Einwegbehälters mit bevorzugter Gestaltung. **2 B** zeigt eine Vorderansicht einer Anordnung von 10 trennbar verbundenen Einwegbehältern, 2 C zeigt eine Seitenansicht und **2 D** eine Draufsicht derselben Anordnung.

### Beispiele

### Beispiel 1: Zusammensetzung einer erfindungsgemäßen Formulierung mit den Wirkstoffen Melatonin, Ginkgo Biloba und Biotin

Die Zusammensetzung enthält 0,05 Gew.-% Ginkgo Biloba Trockenextrakt, 0,01 Gew.-% Biotin, 0,0033 Gew.-% Melatonin sowie weitere Zusatzstoffe, Wasser und Ethanol. Der pH-Wert der Zusammensetzung beträgt zwischen 3,5 und 4.

### Beispiel 2: Klinische Studie

Die in Beispiel 1 beschriebene Zusammensetzung wurde in einer randomisierten Doppelblind-Studie mit Placebo-Kontrolle und einem Crossover-Design an 8 gesunden weiblichen Patienten getestet. Die Tagesdosis war 0,1 mg Melatonin (3 ml Zusammensetzung), die täglich für 14 Tage vor dem Schlafen auf die Kopfhaut aufgetragen wurde.

Urin und Blut der Patientinnen wurden gesammelt und einer Bestimmung von Melatonin bzw. dem Metaboliten 6-Hydroxymelatoninsulfat unterzogen.

Es wurde gefunden, dass die Melatoninplasmaprofile der mit Melatonin oder Placebo behandelten Patientinnen den in der Literatur beschriebenen Konzentration-Zeit-Profilen für endogenes Melatonin entsprachen. Dies belegt, dass die topische Applikation der Melatonin-enthaltenden Zusammensetzung für 14 Tage die endogene Melatoninsekretion nicht verändert.

Die Verabreichung der Zusammensetzung für 14 Tage führte zu keinem Anstieg der Melatoninkonzentration im Plasma bzw. der Konzentration von 6-Hydroxymelatoninsulfat im Urin über physiologische Konzentrationen hinaus. Die topische Applikation der Melatonin-enthaltenden Zusammensetzung war nicht mit Nebenwirkungen verbunden. Es wurden auch keine Wirkungen auf Reaktionszeiten oder kortikale Erregungsparameter gefunden.

Insgesamt ist festzustellen, dass die Verabreichung der Zusammensetzung keine systemischen Wirkungen induziert.

## Patentansprüche

1. Einwegbehälter für ein Arzneimittel oder enthielt, kosmetisches Mittel zur topischen Applikation, enthaltend eine Einzeldosis an Melatonin oder einem Melatonin-Derivat, die einer lokal wirksamen Dosis entspricht, die aber keine systemische Wirkung hervorruft, wobei die Einzeldosis von 0,01 bis 0,2 mg Melatonin oder Melatonin-Derivat beträgt und die Einzeldosis einer Tagesdosis entspricht, wobei das Mittel als Wirkstoffe eine Kombination von Melatonin oder einem Melatonin-Derivat mit Biotin und Gingko Biloba enthält.

2. Behälter nach Anspruch 1,
**dadurch gekennzeichnet,**
**dass** das Mittel als flüssige Formulierung vorliegt.

3. Behälter nach Anspruch 2,
**dadurch gekennzeichnet,**
**dass** die flüssige Formulierung eine Konzentration von 0,001 bis 0,01 % (Gew.) an Melatonin aufweist.

4. Behälter nach Anspruch 3,
**dadurch gekennzeichnet,**
**dass** die flüssige Formulierung eine Konzentration von 0,003 bis 0,004 % (Gew.) an Melatonin aufweist.

5. Behälter nach einem der vorhergehenden Ansprüche,
**dadurch gekennzeichnet,**
**dass** er 2,5 bis 3,5 ml des Mittels enthält.

6. Behälter nach Anspruch 5,
**dadurch gekennzeichnet,**
**dass** er 2,9 bis 3,2 ml des Mittels enthält.

7. Behälter nach einem der vorhergehenden Ansprüche,
**dadurch gekennzeichnet,**
**dass** das Mittel unter GMP-Bedingungen abgefüllt ist.

8. Behälter nach einem der vorliegenden Ansprüche,
**dadurch gekennzeichnet,**
**dass** er aus einem Kunststoff besteht.

9. Behälter nach Anspruch 8,
**dadurch gekennzeichnet,**
**dass** er aus Polyethylen, Polypropylen, Polyvinylchlorid, Polystyrol oder einem Gemisch daraus besteht.

10. Behälter nach einem der Ansprüche 1 bis 9,
**dadurch gekennzeichnet,**
**dass** er farblos ist.

11. Behälter nach einem der Ansprüche 1 bis 9,
**dadurch gekennzeichnet,**
**dass** er farbig ist.

12. Behälter nach einem der Ansprüche 1 bis 11,
**dadurch gekennzeichnet,**
**dass** er transparent ist.

13. Behälter nach einem der Ansprüche 1 bis 11,
**dadurch gekennzeichnet,**
**dass** er nicht transparent ist.

14. Behälter nach einem der vorherigen Ansprüche,
**dadurch gekennzeichnet,**
**dass** er einen durch Drehen zu öffnenden Einwegverschluss an seinem Kopf aufweist.

15. Behälter nach einem der vorherigen Ansprüche,
**dadurch gekennzeichnet,**
**dass** nach Öffnen das Mittel nur bei Anwendung von Druck austritt.

16. Behälter nach einem der vorherigen Ansprüche,
**dadurch gekennzeichnet,**
**dass** er durch Bedruckung oder Prägung beschriftet ist oder/und mit einem Etikett versehen ist.

17. Anordnung von mehreren miteinander trennbar verbundenen Behältern nach einem der Ansprüche 1 bis 16.

18. Anordnung nach Anspruch 17,
**dadurch gekennzeichnet,**
**dass** 5 Behälter miteinander trennbar verbunden sind.

19. Anordnung nach Anspruch 17,
**dadurch gekennzeichnet,**
**dass** 10 Behälter miteinander trennbar verbunden sind.

20. Anordnung nach einem der Ansprüche 17 bis 19,
**dadurch gekennzeichnet,**
**dass** sie in einen Aluminiumbeutel verpackt ist.

21. Anordnung nach Anspruch 20,
**dadurch gekennzeichnet,**
**dass** der Beutel aus einer Aluminium enthaltenden lamierten Folie besteht und zusätzlich Polyethylen, Polyester oder/und Papier umfasst.

22. Anordnung nach Anspruch 20 oder 21,
**dadurch gekennzeichnet,**
**dass** der Aluminiumbeutel bedruckt ist oder/und mit einem Etikett versehen ist.

23. Verpackungseinheit, umfassend mehrere Anordnungen nach einem der Ansprüche 17 bis 22.

## Claims

1. A disposable container which contains a medicament or cosmetic agent for topical application, containing a single dose of melatonin or of a melatonin derivative which corresponds to a locally effective dose but which does not cause any systemic effect, wherein the single dose is 0.01 to 0.2 mg of melatonin or melatonin derivative, and wherein the single dose corresponds to a daily dose, wherein the agent contains, as active substances, a combination of melatonin or a melatonin derivative together with biotin and gingko biloba.

2. The container as claimed in claim 1,
**characterized in that**
the agent is present as a liquid formulation.

3. The container as claimed in claim 2,
**characterized in that**
the liquid formulation has a concentration of 0.001 to 0.01 % (by weight) of melatonin.

4. The container as claimed in claim 3,
**characterized in that**
the liquid formulation has a concentration of 0.003 to 0.004% (by weight) of melatonin.

5. The container as claimed in any one of the preceding claims,
**characterized in that**
it contains 2.5 to 3.5 ml of the agent.

6. The container as claimed in claim 5,
**characterized in that**
it contains 2.9 to 3.2 ml of the agent.

7. The container as claimed in any one of the preceding claims,
**characterized in that**
the agent is filled into the container under GMP conditions.

8. The container as claimed in any one of the preceding claims,
**characterized in that**
it is made of plastic.

9. The container as claimed in claim 8,
**characterized in that**
it is made of polyethylene, polypropylene, polyvinyl chloride, polystyrene or a mixture of these.

10. The container as claimed in any one of claims 1-9,
**characterized in that**
it is colorless.

11. The container as claimed in any one of claims 1-9,
**characterized in that**
it is colored.

12. The container as claimed in any one of claims 1-9,
**characterized in that**
it is transparent.

13. The container as claimed in any one of claims 1-9,
**characterized in that**
it is non-transparent.

14. The container as claimed in any one of the preceding claims,
**characterized in that**
it has, on its head, a disposable closure piece which is opened by turning it.

15. The container as claimed in any one of the preceding claims,
**characterized in that**
after opening, the agent emerges only when pressure is applied.

16. The container as claimed in any one of the preceding claims,
**characterized in that**
it carries lettering either printed on or embossed and/or is provided with a label.

17. An arrangement of several containers, as claimed in any one of claims 1 to 16, which are connected to one another in a detachable manner.

18. The arrangement as claims in claim 17,
**characterized in that**
5 containers are connected to one another in a detachable manner.

19. The arrangement as claims in claim 17,
**characterized in that**
10 containers are connected to one another in a detachable manner.

20. The arrangement as claimed in any one of claims 17 to 19,
**characterized in that**
it is packed into an aluminum bag.

21. The arrangement as claims in claim 20,
**characterized in that**
the bag consists of an aluminum-containing laminated foil and additionally includes polyethylene, polyester and/or paper.

22. The arrangement as claims in claim 20 or 21,
**characterized in that**
the aluminum bag is printed on and/or is provided with a label.

23. A package unit comprising several arrangements as claimed in any one of claims 17 to 22.

## Revendications

1. Conteneur à usage unique, qui contient un médicament ou un agent cosmétique pour application topique, contenant une dose unique de mélatonine ou d'un dérivé de mélatonine qui correspond à une dose efficace localement, mais qui n'entraîne pas d'effet systémique, la dose unique étant de 0,01 à 0,2 mg de mélatonine ou d'un dérivé de mélatonine et la dose unique correspondant à une dose journalière, l'agent contenant comme principe actif, une combinaison de mélatonine ou d'un dérivé de mélatonine avec de la biotine et du gingko biloba.

2. Conteneur selon la revendication 1,
**caractérisé en ce**
**que** l'agent se présente sous la forme d'une formulation fluide.

3. Conteneur selon la revendication 2,
**caractérisé en ce**
**que** la formulation fluide présente une concentration de 0,001 à 0,01 % (en poids) de mélatonine.

4. Conteneur selon la revendication 3,
**caractérisé en ce**
**que** la formulation fluide présente une concentration de 0,003 à 0,004% (en poids) de mélatonine.

5. Conteneur selon l'une des revendications précédentes,
**caractérisé en ce**
**qu'**il contient 2,5 à 3,5 ml de l'agent.

6. Conteneur selon la revendication 5,
**caractérisé en ce**
**qu'**il contient 2,9 à 3,2 ml de l'agent.

7. Conteneur selon l'une des revendications précédentes,
**caractérisé en ce**
**que** l'agent est conditionné dans des conditions de BPF.

8. Conteneur selon l'une des revendications précédentes,
**caractérisé en ce**
**qu'**il est en une matière plastique.

9. Conteneur selon la revendication 8,
**caractérisé en ce**
**qu'**il est en polyéthylène, polypropylène, chlorure de polyvinyle, polystyrène ou en un mélange de ceux-ci.

10. Conteneur selon l'une des revendications 1 à 9,
**caractérisé en ce**
**qu'**il est incolore.

11. Conteneur selon l'une des revendications 1 à 9,
**caractérisé en ce**
**qu'**il est coloré.

12. Conteneur selon l'une des revendications 1 à 11,
**caractérisé en ce**
**qu'**il est transparent.

13. Conteneur selon l'une des revendications 1 à 11,
**caractérisé en ce**
**qu'**il n'est pas transparent.

14. Conteneur selon l'une des revendications précédentes,
**caractérisé en ce**
**qu'**il présente à sa tête, un bouchon à usage unique à ouvrir en le faisant tourner.

15. Conteneur selon l'une des revendications précédentes,
**caractérisé en ce**
**que**, après l'ouverture, l'agent ne sort que par l'application d'une pression.

16. Conteneur selon l'une des revendications précédentes,
**caractérisé en ce**
**qu'**il porte une impression ou une empreinte et/ou est doté d'une étiquette.

17. Dispositif de plusieurs conteneurs associés les uns aux autres de façon séparable, selon l'une des revendications 1 à 16.

18. Dispositif selon la revendication 17,
**caractérisé en ce**
**que** 5 conteneurs sont associés les uns aux autres de façon séparable.

19. Dispositif selon la revendication 17,
**caractérisé en ce**
**que** 10 conteneurs sont associés les uns aux autres de façon séparable.

20. Dispositif selon l'une des revendications 17 à 19,
**caractérisé en ce**
**qu'**il est empaqueté dans un sachet en aluminium.

21. Dispositif selon la revendication 20,
**caractérisé en ce**
**que** le sachet est constitué d'une feuille stratifiée contenant de l'aluminium et comprend en outre du polyéthylène, du polyester et/ou du papier.

22. Dispositif selon la revendication 20 ou 21,
**caractérisé en ce**
**que** le sachet en aluminium porte une impression et/ou est doté d'une étiquette.

23. Unité de conditionnement comprenant plusieurs dispositifs selon l'une des revendication 17 à 22.
